# EUROPEAN PATENT SPECIFICATION

(11) **EP 1 415 633 B1**
(45) Date of publication and mention of the grant of the patent: **10.03.2010**
(21) Application number: 03013548.7
(22) Date of filing: 13.06.2003
(51) Int. Cl.: A61H 39/06, A61H 39/04, A61H 1/00

(54) **Heat therapy system**
Wärmetherapiesystem
Système de thérapie thermique

(30) Priority: 29.10.2002 KR 2002032238; 29.10.2002 KR 2002032239
(43) Date of publication of application: 06.05.2004
(73) Proprietor: Migun Medical Instrument Co., Ltd., Yeongi-gun, Chungcheongnam-do, 339-862 (KR)
(72) Inventor: Park, Mi-Ja, Yeongi-gun, Chungcheongnam-do, 339-862 (KR)
(74) Representative: TER MEER - STEINMEISTER & PARTNER GbR

(56) References cited:
- WO-A-00/56262
- WO-A-01/60310
- WO-A-86/05091
- KR-U- 200 170 080
- US-B1- 6 243 609
- US-B1- 6 454 732
- PATENT ABSTRACTS OF JAPAN vol. 2000, no. 24, 11 May 2001 (2001-05-11) -& JP 2001 198149 A (MIGUN MEDICAL INSTRUMENT CO LTD), 24 July 2001 (2001-07-24)

## Description

### BACKGROUND OF THE INVENTION

### Field of the Invention

The present invention relates to a heat therapy system for applying acupressure and thermal treatment to aching parts of a patient's body using high-temperature heat and far-infrared rays emitted from the heat therapy device, thereby preventing and healing various diseases.

### Description of the Related Art

Generally, the human body has a plurality of vital acupuncture points. These acupuncture points have been widely utilized and developed in folk remedies. Namely, where acupressure and massage treatments are applied on the acupuncture points, cells of that compressed region around the acupuncture points emit heat. At this time, waste matter is discharged therefrom, and consequently the acupuncture points are opened up, thereby achieving a state suitable for certain treatments.

Examples of well known physical therapy apparatuses using the above method generally include a heat therapy apparatus adapted to apply acupressure and thermal treatment to aching parts of the patient's body using a helium lamp, infrared lamp and so on, and a high-frequency therapy apparatus using a high-frequency lamp.

In order to use the above therapy apparatus, the patient, first, has to accurately position the apparatus on the acupuncture points around spinal joints, and to move it to other acupuncture points around next spinal joints at regular time intervals.

Such a therapy method, however, has a problem that it is difficult for motor impairment patients to use the therapy apparatus because the patient has to position the therapy apparatus on the spinal joints by himself/herself, and also move it to the next spinal joints at regular time intervals by himself/herself.

In addition, it is difficult for most patients to accurately position the therapy apparatus on the acupuncture points around the spinal joints. Consequently, most patients cannot effectively use the therapy apparatus.

A heat therapy device of a heat therapy system, designed by recognizing the above problems, is disclosed in a utility model registration registered in the Korean Industrial Property Office as Registration No. 20-0201798. The disclosed heat therapy system is provided with a plurality of acupressure heads, respectively, integrated with far-infrared emitting material. The heat therapy system has a body provided at its bottom surface with heating elements. The heating elements are arranged at equal distances, and, especially, are densely arranged in recessed portions under the respective acupressure members.

In addition, a utility model registration, registered in the Korean Industrial Property Office as Registration No. 20-017 0080, discloses a heat therapy device of a heat therapy system constructed in such a fashion that the middle portion of the heat therapy device is indented at both side surfaces thereof, and handles are provided in the both indented side surfaces of the middle portion, respectively, thereby allowing the patient to easily hold the heat therapy device to aching parts of the his/her body while gripping the handles. This configuration of the heat therapy device enables the healing of the entire body to some extent.

The conventional techniques as stated above, however, have problems that it is impossible to effectively apply the heat therapy device of the heat therapy system to the entire body without restriction, and to simultaneously apply acupressure and thermal treatment to aching parts of the patient's body.

In short, the heat therapy system using the heat therapy device is a physical therapy apparatus for applying acupressure and thermal treatment around the spinal joints of the patient using high-temperature heat and far-infrared rays emitted from the heat therapy device, thereby preventing and healing various diseases. Namely, the heat therapy device horizontally and vertically reciprocates, thereby applying acupressure and thermal treatment around the spinal joints of the patient.

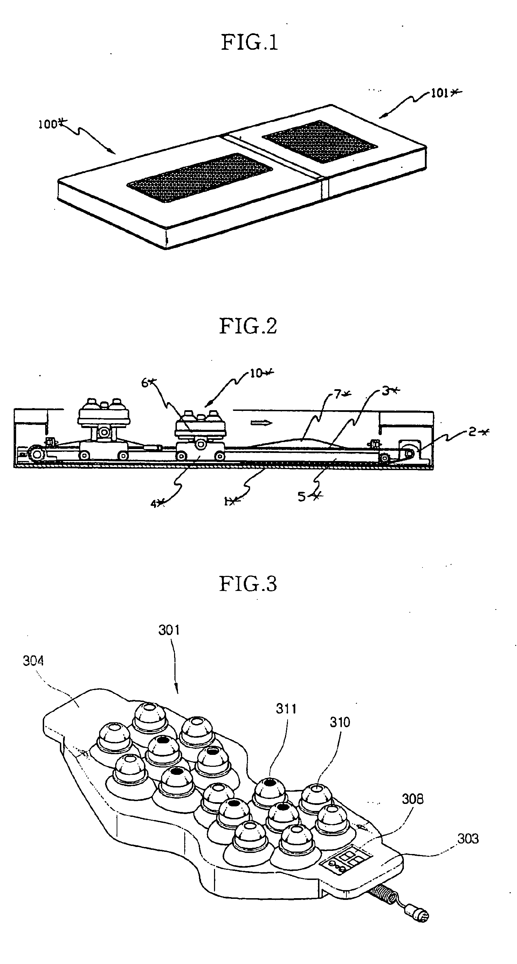

Figs. 1 and 2 is a perspective view and a sectional view, illustrating a conventional heat therapy system. As shown in Figs. 1 and 2, a conventional heat therapy system of bed type, comprises a mattress-shaped body 1*, a motor 2*, a chain 3*, a plurality of carriers 4*, a rail 5*, a plurality of moving plates 6* mounted on the respective carriers 4*, respectively, a curved rail 7*, and plural heat therapy devices 10* mounted on the respective moving plates 6*, respectively. In operation, as the chain 3* is driven by the motor 2*, the carriers 4* linearly reciprocate along the rail 5*. At the same time with the reciprocating movement of the carriers 4*, the moving plates 6* vertically reciprocate along the curved rail 7*.

Considering the external appearance of the conventional heat therapy bed, the heat therapy bed comprises a main mattress 100*, onto which the patient's upper body is laid, and an auxiliary mattress 101*, onto which the patient's lower body is laid. By covering these mattresss with cloth and leather sheets after installing a sponge having a certain thickness on the mattresss, the heat therapy bed is completed. In use, when the patient operates the motor and heat therapy devices using a remote controller in a state wherein the patient lies on the heat therapy bed, the heat therapy devices horizontally and vertically reciprocate, thereby applying acupressure and thermal treatment to the cervical vertebrae, thoracic vertebrae, lumbar vertebrae and so on of the patient.

The conventional heat therapy devices are adapted to vertically reciprocate along the spinal curvature of the patient, lying on the mattresss, thereby applying acupressure to acupuncture points around the spine of the patient, and applying thermal treatment to the acupuncture points using high-temperature heat and far-infrared rays emitted from the heat therapy devices.

As seen from the above description, in order to increase a healing effect, the conventional heat therapy bed has a relatively complex structure with a plurality of the carriers driven by the motor to horizontally reciprocate, and a plurality of the heat therapy devices installed on the respective carriers, respectively, to apply acupressure and thermal treatment to aching parts of the patient. That is to say, in order to install a plurality of the heat therapy devices for increasing effects of acupressure and thermal treatment, the conventional heat therapy bed is installed with a plurality of the carriers. This deteriorates operability and productivity thereof, as well as increasing its manufacturing cost.

In order to solve the above problems, a utility model registration, registered in the Korean Industrial Property Office as Registration No. 20-0288224. discloses a device for vertically reciprocating heat therapy devices. The device is configured in such a fashion that a carrier, driven by a motor to horizontally reciprocate, is installed at its both sides with brackets adapted to rotate by hinge shafts and link bars, and the both brackets are installed with a pair of heat therapy devices, respectively, thereby increasing effects of acupressure and thermal treatment to the acupuncture points. In addition, the device is constructed to ensure smooth operation of the carrier and heat therapy devices, thereby improving productivity and reducing a manufacturing cost.

The device for vertically operating the heat therapy devices, however, has problems that it cannot adjust the strength of acupressure and thermal treatment according to the different body shape and aching parts of the patient, and that the heat therapy devices coupled to the carrier provided in a lower mattress cannot move according to the motion of the patient's legs, thereby deteriorating a heat therapy effect. In addition, in the case of the device's failure, the patient has to disassemble the device to repair it.

WO 00/56262 A1 describes a finger-pressure fomentation device. Herein, a portable pressure and fomentation device comprises a body, which has a flat top that is sized to allow a user to lay his back on the flat top while he is in a reclined position. A plurality of pressing cones are respectively engaged with through holes formed through the top of the body to be projected upward. Herein, the pressing cones mounted to the body are situated to be able to press two side acupressure regions that are respectively spaced apart from the vertebra by a certain distance. In addition, armrests are provided on which the user rests his arms and which are respectively extended from the side ends of the body. Further, an operating switch for a controller, a power switch, a temperature increasing button, a temperature reducing button, a LCD for displaying a set temperature, and a LCD for displaying an actual temperature are mounted to the outside of the body. The pressing cones generate not only an acupressure effect and a fomentation effect but also a far infrared ray emitting effect beneficial to the human body. Further, the pressing cones may be made of metal that is good in heat conductivity. Additionally, the pressing cones may be double-layered cones that are respectively made of metal and jade.

US 6,454,732 B1 describes an apparatus for rising and falling medicator of automatic hot-heat treatment device. Herein, a hot-heat treatment device includes a mainbody in a mat type on which a user is to lie down. A reciprocal motor is fixedly installed at a portion of the main body. A screw is rotated upon receipt of a driving force by a belt as the reciprocal motor is driven. Further, a movable body being threaded with the screw is reciprocally moved forwardly and backwardly as the screw rotates. The movable body is connected with a treatment device moving plate having a moving bar at its lower portion to be inserted in a space of the movable body and movement rails at its right and left sides. In addition, an indented rail has an indented surface, on which the treatment device moving plate having a medicator mounted thereon is moved upwardly and downwardly, when the movable body is moved forwardly and backwardly.

US 6,243,609 B1 describes a treatment mat. Herein, a mat is foldable at the one side thereof and centrally provided with a longitudinal groove. In this groove, a hyperthermo-therapeutical apparatus is moving along a rail. In addition, a conveyer for moving the hyperthermo-therapeutical apparatus is provided, wherein the conveyer is controlled by a control panel. The position of the hyperthermo-therapeutical apparatus is controlled by a control unit. Further, a pair of wheels are provided on both lateral sides of the hyperthermo-therapeutical apparatus to be moved along the rail provided in the longitudinal groove of the mat. In addition, a motor for driving the conveyer in accordance with a signal output from the control panel and a wire connecting a pulley at the motor side to an idle pulley at the other side of the mat are provided, wherein the end of the wire is associated with the hyperthermo-therapeutical apparatus. The hyperthermo-therapeutical apparatus applies a hot compress to the vertebral acupuncture point of a patient and simultaneously a far infrared radiation.

WO 01/60310 A1 describes a spine massager. Herein, a spine massager includes a base body, and a carrier mounted in a recess formed at the central portion of the base body in such a fashion that it can reciprocate longitudinally. A massaging member having a plurality of massaging protrusions is mounted on the carrier. A lamp for generating far infrared rays and electrical heat is contained in the massaging member. A driving mechanism of the spine massager includes a rack gear provided at the bottom surface of the recess of the base body, a pinion gear mounted to the lower portion of the carrier and a drive motor fixedly mounted to the carrier and adapted to transmit a driving force to the pinion gear. The rotating force of the drive motor is transmitted to the pinion gear by a power transmission means, so that the carrier is horizontally movable in a desired direction along the rack gear formed at the base body. Thus, the carrier reciprocates in accordance with normal and reverse rotations of the drive motor.

### SUMMARY OF THE INVENTION

Therefore, the present invention has been made in view of the above problems, and it is an object of the present invention to provide a heat therapy system comprising a heat therapy device, which is configured in such a fashion that it simultaneously applies acupressure and thermal treatment to aching parts of the patient's body and may be effectively used on any regions of the patient's body without restriction, thereby maximizing a healing effect of the system and allowing it to be widely used on the patient's entire body. This object is solved by a heat therapy system according to claim 1. Further advantages, refinements and embodiments of the invention are described in the respective sub-claims.

The heat therapy system of the present invention comprises a heat therapy device configured in such a fashion that it is provided at the upper surface thereof with a plurality of upwardly-protruded acupressure nobs, to which superconductive and far-infrared emitting material is attached, respectively, and both side portions of the device are curved upwardly and are provided with handles, respectively.

In addition, the heat therapy system of the present invention further comprises upper and lower bodies; control boxes positioned at adjacent portions of the upper and lower bodies and adapted to control them, respectively; upper control panels connected to the upper side of the respective control boxes, respectively; motors installed, respectively, at the lower portion of the respective upper and lower bodies; respective pulleys installed opposite to the respective motors; upper and lower rails installed between the motors and pulleys; timing belts located between the upper and lower rails while being directly connected to the motors and pulleys; upper and lower mobile units seated on the upper and lower rails, respectively, while being coupled with the respective timing belts, each of the mobile units being formed with a plurality of heat therapy devices; and additional heat therapy devices provided at right and left sides of the upper rails.

According to the present invention, by virtue of the superconductive an far-infrared emitting material attached to the respective acupressure nobs, it is possible to reduce a heat generating time of the acupressure nobs as well as to emit a large amount of far-infrared rays therefrom, thereby enhacing a healing effect of the heat therapy device and the heat therapy system using the same.

It is an advantage of the present invention to enable the patient to adjust the strength of acupressure and thermal treatment according to the different body shape and aching parts of the patients, thereby preventing the deterioration of a thermal treatment effect thereof due to the different body shapes of the patients. The heat therapy system according to the present invention comprises a portable heat therapy device installed to apply acupressure on the front part of the patient lying on his/her back on the heat therapy system for applying acupressure on the body using the heat therapy devices.

### BRIEF DESCRIPTION OF THE DRAWINGS

The above and other objects, features and other advantages of the present invention will be more clearly understood from the following detailed description taken in conjunction with the accompanying drawings, in which:
Fig. 1 is a perspective view illustrating a conventional heat therapy system;
Fig. 2 is a sectional view illustrating an installed state of the conventional heat therapy system;
Fig. 3 is a perspective view illustrating a heat therapy device of a heat therapy system in accordance with the present invention;
Figs. 4a and 4b are a plan view and side view, respectively, illustrating the heat therapy device of the heat therapy system according to the present invention;
Fig. 5 is a plan view illustrating the heat therapy system in accordance with the present invention;
Fig. 6 is a side view illustrating the heat therapy system according to the present invention;
Fig. 7 is a side view illustrating upper rails in accordance with the present invention;
Fig. 8 is a perspective view illustrating an upper mobile unit in accordance with the present invention;
Fig. 9 is an exploded view illustrating a tensile force adjustment device in accordance with the present invention;
Fig. 10 is a plan view illustrating an installed state of the tensile force adjustment device according to the present invention;
Fig. 11 is a perspective view illustrating a lower mobile unit in accordance with the present invention;
Fig. 12 an exploded view illustrating the lower mobile unit of the present invention;
Fig. 13 is a view illustrating the operation of the upper mobile unit according to the present invention; and
Fig. 14 is a plan view illustrating the operation of the lower mobile unit according to the present invention.

### DESCRIPTION OF THE PREFERRED EMBODIMENTS

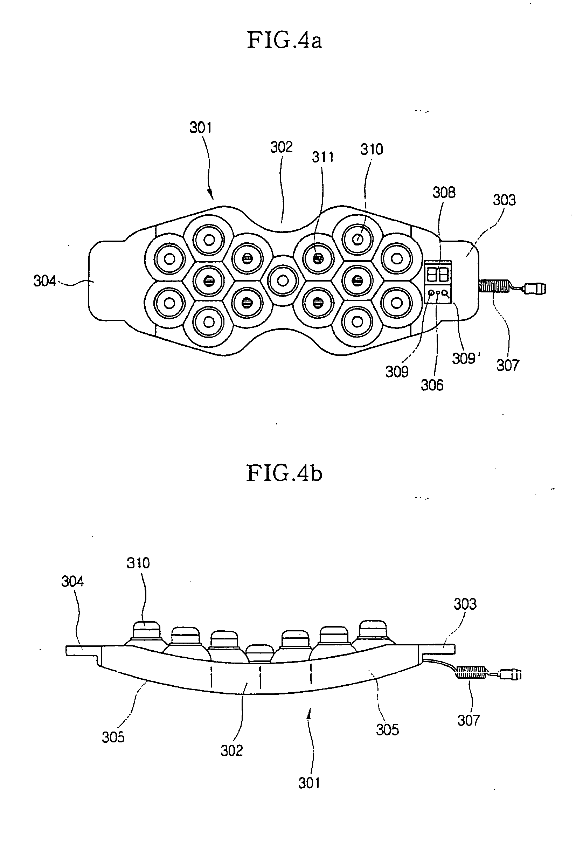

Fig. 3 is a perspective view illustrating a heat therapy device of a heat therapy system in accordance with the present invention. Figs. 4a and 4b are a plan view and side view, respectively, illustrating the heat therapy device of the heat therapy system according to the present invention. As shown in Figs. 3 to 4b, the heat therapy device, designated as a reference numeral 301, comprises a middle portion 302, handles 303 and 304, both end portions 305, an ON/OFF button 306, an electric power line 307, a temperature display window 308, upper and lower temperature adjustment buttons 309 and 309', acupressure nobs 310, and superconductive and far-infrared emitting material 311.

Considering the structure of the heat therapy device 301 in detail, it is provided at its upper surface with a plurality of the upwardly-protruded acupressure nobs 310, and each of the acupressure nobs 310 is attached with the superconductive and far-infrared emitting material 311. The middle portion 302 of the heat therapy device 301 is indented, and the both side portions 305 thereof are curved upwardly at a constant angle to allow them to come into close contact with certain regions, especially, the back or abdomen, of the patient.

The handles 303 and 304 are formed at the upper ends of both side surfaces of the heat therapy device 301, respectively. The digital temperature display window 308 is installed at one side of the upper surface of the heat therapy device 301, and the upper and lower temperature adjustment buttons 309 and 309' are installed at one side of the temperature display window 308.

The ON/OFF button 306, of toggle type, is provided between the upper and lower temperature adjustment buttons 309 and 309', and the electric power line 307 is drawn from one side surface of the heat therapy device 301 under one of the handles 303.

Now, the method of using the heat therapy device constructed as stated above is described. First, the electric power line 307 is connected to a power supply, and then the temperature of the heat therapy device 301 is appropriately adjusted using the temperature adjustment buttons 309 and 309'. After that, as the patient brings the heat therapy device 301 into contact with aching parts of the body while gripping the handles 303 and 304, the acupressure nobs 310 come into close contact with corresponding acupuncture points of the body, thereby applying acupressure and thermal treatment to the body.

In operation, by virtue of the superconductive and far-infrared emitting material 311 attached in the respective acupressure nobs 310, a heat generating time of the acupressure nobs 310 is reduced and a large amount of far-infrared rays is emitted, thereby increasing a healing effect thereof.

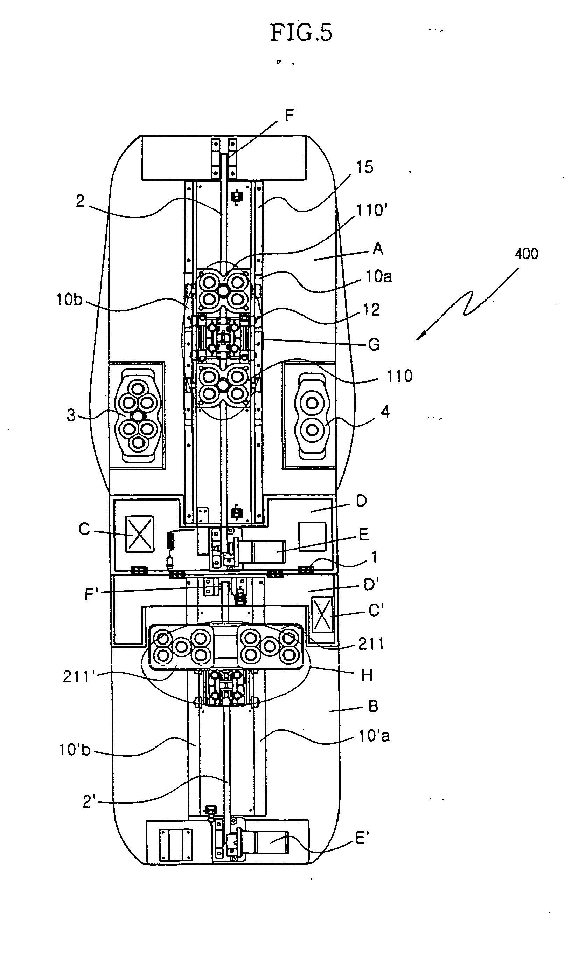

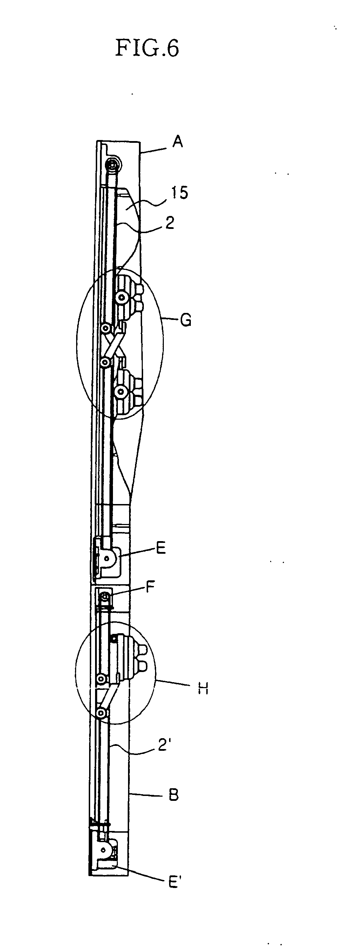

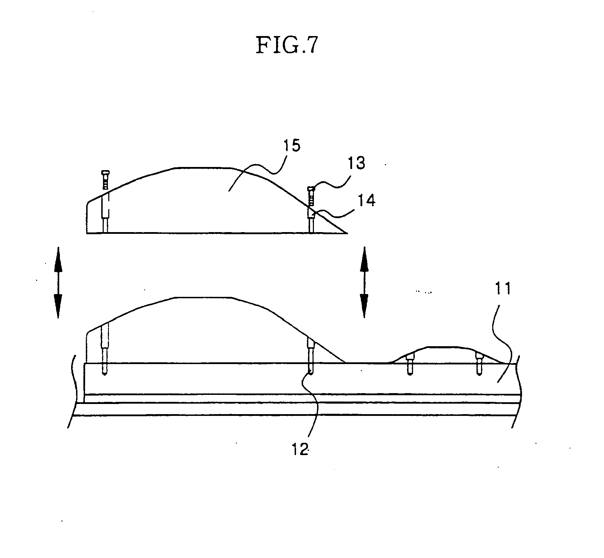

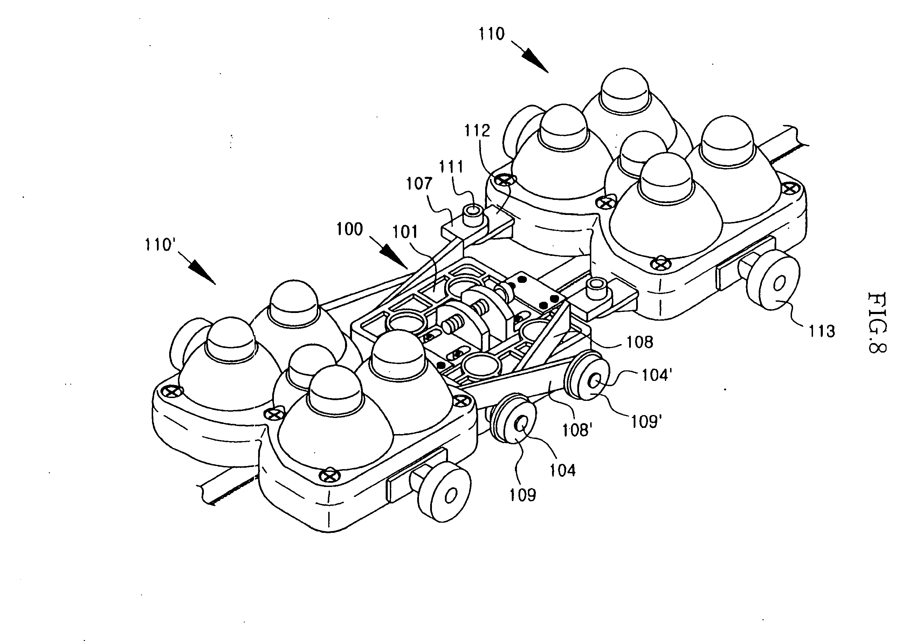

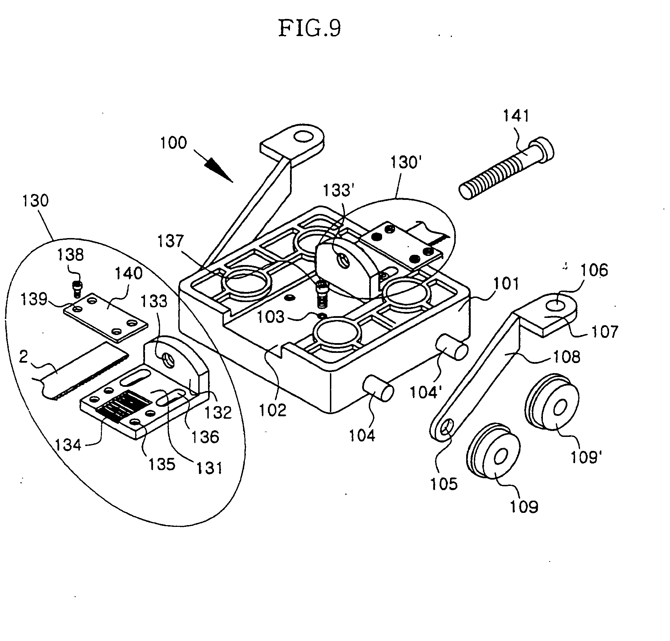

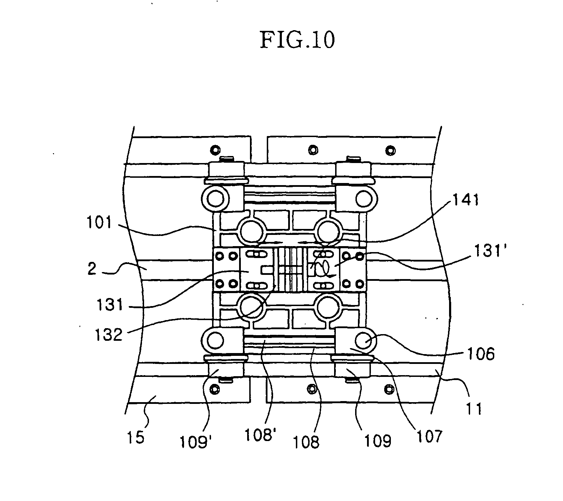

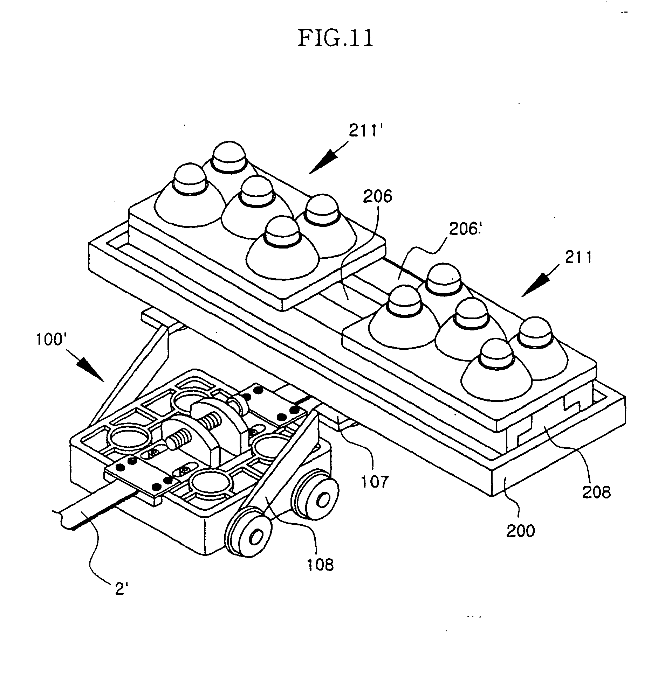

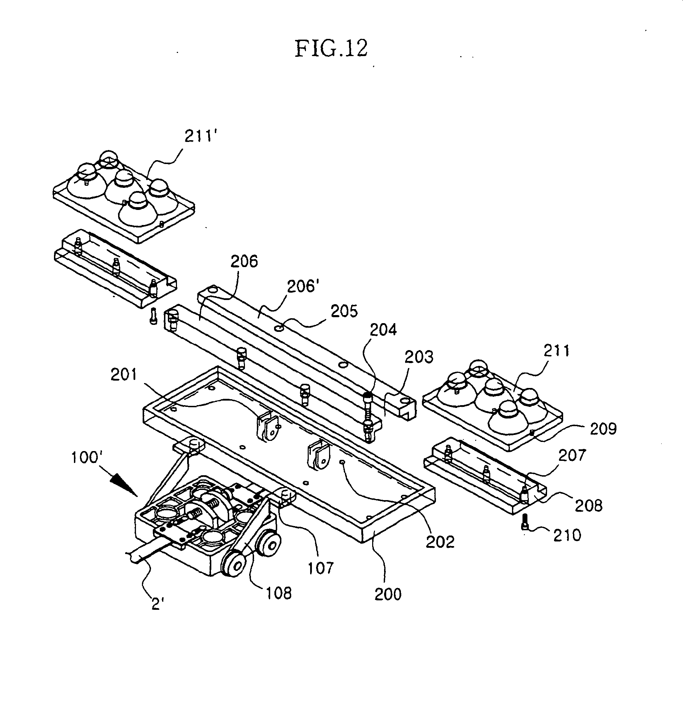

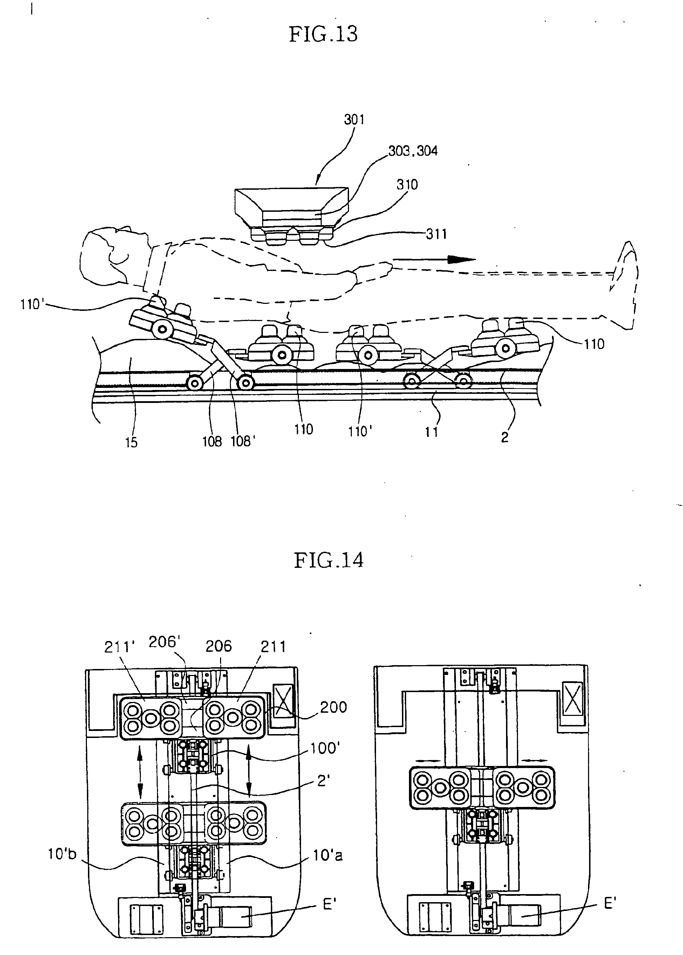

Figs. 5 to 14 are views illustrating a heat therapy system in accordance with the present invention. As shown in Figs. 5 to 14, the heat therapy system in accordance with the present invention, designated as a reference numeral 400, comprises connectors 1, upper and lower timing belts 2 and 2', heat therapy devices 3, 4, 110, 110', 211 and 211', upper rails 10a and 10b, lower rails 10'a and 10'b, auxiliary rails 11, 206 and 206', screw holes 12, 14, 133 and 133', screws 13, 137, 138, 204 and 210, perforated holes 105, 106, 139 and 205, curved rail plates 15, carriers 100 and 100', a body 101, a coupling groove 102, screw bores 103, 202 and 209, hinges 104 and 104', coupling portions 107 and 112, link bars 108 and 108', rollers 109, 109', 113 and 201, a protrusion 111, a bracket 200, a T-shaped groove 203, a T-shaped insertion member 208, a first tensile force adjustor 130, a second tensile force adjustor 130', a lower tensile force adjustment plate 131, an upper tensile force adjustment plate 132, belt fastening slits 134, fixing holes 135, movement slots 136, an upper fixing plate 140, a tensile force adjustment screw 141, an upper body A, a lower body B, control boxes C and C', upper control panels D and D', motors E and E', pulleys F and F', an upper mobile unit G, a lower mobile unit H and a tensile force adjustment device I. The heat therapy system 400 is completed by adding the heat therapy device 301.

Explaining the construction of the heat therapy system 400 in detail, the heat therapy system 400 comprises the upper and lower bodies A and B defining a receiving space therein, respectively. The upper and lower bodies A and B are provided with the control boxes C and C' at adjacent portions thereof, which are adapted to control respective components of the upper and lower bodies A and B, respectively. The respective control boxes C and C' are connected at their upper sides to the control panels D and D', having a "U-shaped" plate shape, through the connectors 1. This configuration of the control panels D and D' facilitate after-service of the control boxes C and C'. The upper and lower bodies A and B are provided with the motors E and E' at the center positions of the their respective lower portions, the pulleys F and F' at the opposite sides of the motors E and E', and upper and lower rails 10a, 10b, 10'a and 10'b installed between the motors E and E' and pulleys F and F', respectively. The upper and lower rails 10a, 10b, 10'a and 10'b are arranged at both sides to correspond to the patient's spine. Between the upper and lower rails 10a, 10b, 10'a and 10'b, the timing belts 2 and 2' are positioned along a central axis therebetween in a state wherein they are directly connected to the motors E and E' and pulleys F and F', respectively. Each timing belt 2 or 2' is formed with bosses at its lower surface. The heat therapy system 400 comprises the upper and lower mobile units G and H, which are coupled with the timing belts 2 and 2', respectively, and seated on the upper and lower rails 10a, 10b, 10'a and 10'b. The upper and lower mobile units G and H are formed with a plurality of the heat therapy devices 110, 110', 211 and 211'. In addition to the heat therapy devices 110, 110', 211 and 211', other two heat therapy devices 3 and 4 are formed on the upper body A, respectively. The heat therapy devices 3 and 4 are formed at left and right sides of the upper rails 10a and 10b and adapted to apply acupressure and thermal treatment to both arms of the patient.

Each of the upper rails 10a and 10b comprises the rail 11 formed at its upper portion with a plurality of the screw holes 12, and a plurality of the detachable curved rail plates 15 formed at their upper portions with a plurality of the screw holes 14, respectively. The curved rail plates 15 are arranged at regular intervals. Through the screw holes 14 of the curved rail plates 15, screws 13 are fastened to the upper portion of the rail 11.

The upper mobile unit G comprises the carrier 100 having a tensile force adjustment device I at a central portion thereof. The carrier 100 has two pairs of the link bars 108 and 108' formed with the coupling portions 107 at respective one side. The coupling portions 107 are adapted to be coupled with the coupling portions 112 of the upper mobile unit G, respectively. Each coupling portion 112 is formed with the upwardly extended protrusion 111 at its upper surface. The upper mobile unit G further has the heat therapy devices 110 and 110' formed with a pair of the rollers 113 at both side surfaces.

The upper carrier 100 comprises the square body 101 formed at a center portion of its upper surface with the linear coupling groove 102 and a pair of the screw bores 103. The tensile force adjustment device I is coupled to the upper side of the coupling groove 102 of the body 101 and adapted to adjust a tensile force of the timing belt 2. The body 101 is formed at both side surfaces thereof with two pairs of hinges 104 and 104'. Each of the link bars 108 and 108' is formed at both end portions with the perforated holes 105 and 106, and at its one end with the coupling portion 107. One of the perforated holes 106 is formed at the coupling portion 107, and the other perforated hole 105 is used for insertion of the hinge 104 or 104'. The respective link bars 108 and 108' are formed with the rollers 109 and 109' at opposite outer sides thereof. The rollers 109 and 109' are adapted to be coupled around the respective hinges 104 and 104', respectively.

The tensile force adjustment device I comprises the first tensile force adjustor 130 formed at one side of the body 101, and the second tensile force adjustor 130' formed at the other side of the body 101. The second tensile force adjustor 130' has the same structure as that of the first tensile force adjustor 130. In addition, the tensile force adjustment device I comprises the tensile force adjustment screw 141 fastened into the screw holes 133 and 133' formed at the first and second tensile force adjustors 130 and 130' and adapted to adjust a tensile force of the timing belt 2.

The first tensile force adjustor 130 is provided with the lower tensile force adjustment plate 131, and the upper tensile force adjustment plate 132. The lower tensile force adjustment plate 131 is coupled to one side of the upper center portion of the square body 101. The upper tensile force adjustment plate 132 is extended upwardly from one edge of the lower tensile force adjustment plate 131 and formed with the tensile force adjustment screw hole 133. The lower tensile force adjustment plate 131 is formed at one side of the upper center portion thereof with the belt fastening slits 134, which are adapted to fasten one of the timing belts 2. A plurality of the fixing holes 135 are formed at both sides of the belt fastening slits 134, and the movement slots 136 are formed adjacent to the fixing holes 135. The movement slots 136, especially, are arranged to correspond with a pair of the screw bores 103 formed at the body 101, thereby allowing the screw 137 to be fastened therethrough. The first tensile force adjustor 130 is also provided with the upper fixing plate 140 for preventing secession of the timing belt 2. The upper fixing plate 140 is formed with a plurality of the perforated holes 139 for allowing the screws 138 to be fastened to the fixing holes 135 therethrough.

By fastening or unfastening the tensile force adjustment screw 141, coupled into the screw holes 133 and 133' formed at the first and second tensile force adjustors 130 and 130', the respective lower tensile force adjustment plates 131 and 131' of the first and second tensile force adjustors 130 and 130' are moved in leftward and rightward directions, thereby adjusting the tensile force of the timing belt 2.

Meanwhile, the lower mobile unit H comprises the carrier 100' having the same structure as that of the carrier 100 of the upper mobile unit G, and a rectangular bracket 200 having upwardly-protruded portions of a certain length. The upwardly-protruded portions are coupled with the coupling portions 107 formed at the respective link bars 108 of the carrier 100', respectively. The bracket 200 is formed at the lower surface thereof with a pair of the rollers 201, and at both sides of the upper surface thereof with a plurality of the screw bores 202 linearly arranged. The rails 206 and 206', having a "L-shaped" cross section, are coupled to the upper side of the screw bores 202 to form a T-shaped groove 203. Each rail 206 or 206' is formed with the perforated holes 205 for allowing screws 204 to be fastened to the screw bores 202 therethrough. The T-shaped insertion member 208 is fitted into the T-shaped groove 203 defined between the rails 206 and 206'. The T-shaped insertion member 208 is formed with a plurality of the bores 207 linearly arranged along a central axis thereof. The lower mobile unit H is further provided with the heat therapy devices 211 and 211' disposed above the T-shaped groove 203. The heat therapy device 211 and 211' are formed with a plurality of the screw bores 209 corresponding to the bores 207, thereby allowing the screws 210 to be fastened therethrough.

According to the construction of the heat therapy system 400 as stated above, one mobile unit, adapted to horizontaly reciprocate by the driving of the motor, is installed with a plurality of the heat therapy devices. The carrier 100, coupled with the timing belt 2, linearly reciprocates along the upper rail 11 by the driving of the motor E, and the heat therapy devices 110 and 110', rotatably coupled to the link bars 108 and 108' of the carrier 100, move in a vertical direction as it pass over the curved rail plates 15. That is to say, the heat therapy devices 110 and 100', coupled to the carrier 100, horizontally reciprocate, thereby applying acupressure and thermal treatment to the acupuncture points around the spine of the patient.

In addition, the operation of the lower mobile unit H formed at the lower body B is described. In the same manner as the carrier 100 of the upper body A, the carrier 100', coupled with the timing belt 2', linearly reciprocates along the lower rails 10'a and 10'b by the driving of the motor E', and the heat therapy devices 211 and 211', coupled to the rails 206 and 206', move in leftward and rightward directions according to the leftward and rightward movements of the lower body of the patient. As stated above, the rails 206 and 206' define the T-shaped groove 203 therebetween, and the T-shaped groove 203 is formed above the bracket 200 rotatably coupled to the link bars 108 of the carrier 100'.

In operation, the heat therapy devices 211 and 211' move in leftward and rightward directions according to the lower body of the patient while horizontally reciprocating by the motor E', thereby effectively applying acupressure and thermal treatment to the lower body of the patient.

Each of the heat therapy devices 110 and 110' installed on the upper mobile unit G and heat therapy device 211 and 211' installed on the lower mobile units H is provided at its upper surface with a plurality of upwardly-protruded acupressure nobs 310, which are attached with the superconductive and far-infrared emitting material 311, respectively. In addition, the respective heat therapy devices 110, 110', 211 and 211' are curved upwardly at a constant angle to allow them to come into close contact with the body, especially the back or abdomen, of the patient. This maximizes a far-infrared emission effect and enables the heat therapy devices to more closely contact the patient's body, thereby improving a healing effect thereof.

Each of the heat therapy devices 3 and 4 installed at the right and left sides of the upper rails 10a and 10b is provided at its upper surface with a plurality of upwardly-protruded acupressure nobs 310, which are attached with the superconductive and far-infrared emitting material 311, respectively. In addition, the heat therapy devices 3 and 4 are curved upwardly at a constant angle to allow them to come into close contact with the body, especially the back or abdomen, of the patient. This maximizes a far-infrared emission effect and enables the heat therapy devices to more closely contact the patient's body, thereby improving a healing effect thereof.

As apparent from the above description, since the plural curved rail plates 15 are adapted to be selectively attached or detached to or from the upper surfaces of the upper rails 10a and 10b, it is possible to adjust the strength of acupressure and thermal treatment according to the different shape and aching parts of the patient's body. As stated above, the upper mobile unit G is adapted to move in a vertical direction according to the curved rail plates 15 and reciprocate linearly along the rail 11 by the motor E, and the heat therapy devices 110 and 110' are coupled to the upper mobile unit G. According to the present invention, the heat therapy devices 110 and 110' are constructed to achieve a maximum height with a minimum volume. This prevents a thermal treatment effect of the devices from deteriorating due to the different body shapes of patients, as well as ensuring the smooth operation thereof. Further, according to the present invention, the lower mobile unit H, adapted to linearly reciprocate along the lower rails 10'a and 10'b by the motor E', is formed at its upper surface with a pair of the heat therapy devices 211 and 211', which are adapted to move in leftward and rightward directions according to the movements of the patient. This prevents the deterioration of a thermal treatment effect of the devices due to the movements of the lower body. Furthermore, by virtue of the "⊂-shaped" upper control panels D and D', provided on the upper side of the control boxes C and C' and constructed into a swing type, the control boxes C and C' can be easily repaired without disassembling the upper and lower bodies A and B.

The heat therapy device of the present invention can apply acupressure and thermal treatment to aching parts of the patient. Further, by virtue of the superconductive and far-infrared emitting material 311 attached in the respective acupressure nobs 310, it is possible to reduce a heat generating time of the acupressure nobs and to emit a large amount of far-infrared rays, thereby enhancing a healing effect thereof.

The heat therapy device 301 is configured so that the both end portions thereof are curved upwardly at a certain angle, thereby enabling it to be widely applied on the entire body of the patient, unlike any conventional heat therapy systems.

## Claims

1. A heat therapy system (400) comprising heat therapy devices, wherein one heat therapy device (301) comprises:
- a plurality of upwardly-protruded acupressure nobs (310) at its upper surface, each of the acupressure nobs (310) being attached with superconductive and far-infrared emitting material (311);
- an indented middle portion (302);
- both side portions (305) being curved upwardly to allow the upwardly-protruded acupressure nobs (310) to come into close contact with a patient's body, especially, the back or abdomen of the patient;
- handles (303, 304) formed at respective upper ends of both side surfaces of the heat therapy device (301), respectively;
- a digital temperature display window (308) installed at one side of an upper surface of the heat therapy device (301);
- upper and lower temperature adjustment buttons (309, 309') installed, respectively, at one side of the temperature display window (308);
- an ON/OFF button (306), of toggle type, provided between the upper and lower temperature adjustment buttons (309, 309'); and
- an electric power line (307) formed at one side surface of the heat therapy device (301) under one of the handles (303) of the heat therapy device (301); the heat therapy system further comprising:
a) upper and lower bodies (A, B) defining a receiving space therein, respectively;
b) control boxes (C, C') formed at adjacent portions of the upper and lower bodies (A, B) and adapted to control respective components, respectively;
c) control panels (D, D') having a U-shaped plate shape and formed into a swing manner such that each control panel (D, D') is connected at its one side to an upper side of the respective control boxes (C, C') through connectors (1), the control panels (D, D') being connected with electric power lines of the heat therapy devices (3, 4, 110, 110', 211, 211');
d) motors (E, E') formed at a center position of respective lower portions of the upper and lower bodies (A, B), respectively;
e) pulleys (F, F') formed at opposite sides of the motors (E, E'), respectively;
f) upper and lower rails (10a, 10b, 10'a, 10'b) installed between the motors (E, E') and pulleys (F, F'), respectively, the upper and lower rails (10a, 10b, 10'a, 10'b) being arranged in two rows to correspond to the spine of the body;
g) timing belts (2, 2') positioned along a central axis between the upper and lower rails (10a, 10b, 10'a, 10'b) while being directly connected to the motors (E, E') and pulleys (F, F'), respectively, each timing belt (2, 2') being formed with bosses at its lower surface;
h) upper and lower mobile units (G, H) coupled with the timing belts (2, 2'), respectively, and seated on the upper and lower rails (10a, 10b, 10'a, 10'b), the upper and lower mobile units (G, H) being formed with a plurality of heat therapy devices (110, 110', 211, 211'); and
i) other two heat therapy devices (3, 4) formed on the upper body (A), the other two heat therapy devices (3, 4) being formed at left and right sides of the upper rails (10a, 10b) and adapted to apply acupressure and thermal treatment to both arms of the patient.

2. The heat therapy system as set forth in claim 1, wherein each of the upper rails (10a, 10b) comprises a rail (11) formed at its upper portion with a plurality of screw holes (12), and a plurality of detachable curved rail plates (15) arranged at regular distances and formed at their upper portions with a plurality of screw holes (14), respectively,
whereby screws (13) are fastened to the upper portion of the rail (11) through the screw holes (14) of the rail plates (15), respectively.

3. The heat therapy system (400) as set forth in claim 1, wherein the upper mobile unit (G) comprises:
- a carrier (100) having a tensile force adjustment device at a central portion thereof;
- coupling portions (112) adapted to be coupled with coupling portions (107) formed at respective one side of two pairs of link bars (108, 108') provided in the carrier (100), respectively, each coupling portion (112) being formed with an upwardly extended protrusion (111) at its upper surface; and
- the heat therapy devices (110, 110') formed with a pair of rollers (113) at both side surfaces, respectively, the carrier (100) comprising:
-- a square body (101) formed at a center portion of its upper surface with a linear coupling groove (102) and a pair of screw bores (103);
-- a tensile force adjustment device coupled to the coupling groove (102) of the body (101) and adapted to adjust a tensile force of one of the timing belts (2);
-- two pairs of hinges (104, 104') formed at both side surfaces of the body (101);
-- link bars (108, 108'), each link bar (108, 108') being formed at both end portions with perforated holes (105, 106) and at its one end with the coupling portion (107), one of the perforated holes (106) being formed at the coupling portion (107), the other perforated hole (105) being used for insertion of the hinge (104, 104'); and
-- rollers (109, 109') formed at opposite outer sides of the respective link bars (108, 108') and adapted to be coupled around the respective hinges (104, 104'), respectively.

4. The heat therapy system as set forth in claim 3, wherein the tensile force adjustment device comprises:
- a first tensile force adjustor (130) formed at one side of the body (101);
- a second tensile force adjustor (130') formed at the other side of the body (101), the second tensile force adjustor (130') having the same structure as that of the first tensile force adjustor (130); and
- a tensile force adjustment screw (141) fastened into the screw holes (133, 133') formed at the first and second tensile force adjustors (130, 130') and adapted to adjust a tensile force of the timing belt (2),
the first tensile force adjustor (130) comprising:
-- a lower tensile force adjustment plate (131) coupled to one side of an upper center portion of the square body (101);
-- an upper tensile force adjustment plate (132) extended upwardly from one edge of the lower tensile force adjustment plate (131) and formed with the tensile force adjustment screw hole (133);
-- belt fastening slits (134) formed at one side of an upper center portion of the lower tensile force adjustment plate (131) and adapted to fasten one of the timing belts (2);
-- a plurality of fixing holes (135) formed at both sides of the belt fastening slits (134);
-- movement slots (136) adjacent to the fixing holes (135), the movement slots (136) being positioned to correspond with a pair of the screw bores (103) formed at the body (101), thereby allowing screws (137) to be fastened therethrough; and
-- an upper fixing plate (140) for preventing secession of the timing belt (2), the upper fixing plate (140) being formed with a plurality of perforated holes (139) for allowing screws (138) to be fastened to the fixing holes (133) therethrough.

5. The heat therapy system as set forth in claim 1, wherein the lower mobile unit (H) comprises:
- a carrier (100') the carrier (100') comprising:
-- a square body (101) formed at a center portion of its upper surface with a linear coupling groove (102) and a pair of screw bores (103);
-- a tensile force adjustment device coupled to the coupling groove (102) of the body (101) and adapted to adjust a tensile force of one of the timing belts (2);
-- two pairs of hinges (104, 104') formed at both side surfaces of the body (101);
-- link bars (108, 108'), each link bar (108, 108') being formed at both end portions with perforated holes (105, 106) and at its one end with the coupling portion (107), one of the perforated holes (106) being formed at the coupling portion (107), the other perforated hole (105) being used for insertion of the hinge (104, 104'); and
-- rollers (109, 109') formed at opposite outer sides of the respective link bars (108, 108') and adapted to be coupled around the respective hinges (104, 104'), respectively;
- a rectangular bracket (200) having upwardly-protruded portions of a certain length, the upwardly-protruded portions being coupled with the coupling portions (107) formed at the respective link bars (108) of the carrier (100') provided in the lower mobile unit;
- a pair of rollers (201) formed at a lower surface of the bracket (200);
- a plurality of screw bores (202) arranged linearly at both sides of an upper surface of the bracket (200);
- rails (206, 206') having a L-shaped cross section, the rails (206, 206') being coupled to an upper side of the screw bores (202) to form a T-shaped groove (203), each rail (206, 206') being formed with perforated holes (205) for allowing screws (204) to be fastened to the screw bores (202) therethrough;
- a T-shaped insertion member (208) adapted to be fitted into the T-shaped groove (203) defined between the rails (206, 206'), the T-shaped insertion member (208) being formed with a plurality of bores (207) linearly arranged along a central axis thereof; and
- heat therapy devices (211, 211') disposed above the T-shaped groove (203) and formed with a plurality of screw bores (209) corresponding to the bores (207), thereby allowing screws (210) to be fastened therethrough.

6. The heat therapy system (400) as set forth in claim 1, wherein each of the heat therapy devices (110, 110', 210. 210') installed on the upper and lower mobile units (G, H) is provided at its upper surface with a plurality of upwardly-protruded acupressure nobs (310), which are attached with the superconductive and far-infrared emitting material (311), respectively; and
wherein the respective heat therapy devices (110, 110', 210, 210') are curved upwardly to allow them to come into close contact with the patient's body, especially the back or abdomen of the patient.

7. The heat therapy system as set forth in claim 1, wherein each of the heat therapy devices (3, 4) installed at left and right sides of the upper rails (10a, 10b) is provided at its upper surface with a plurality of upwardly-protruded acupressure nobs (310), which are attached with the superconductive and far-infrared emitting material (311), respectively; and
- wherein the respective heat therapy devices (3, 4) are curved upwardly to allow them to come into close contact with the body, especially the back or abdomen, of the patient.

## Patentansprüche

1. Wärmetherapiesystem (400), das Wärmetherapievorrichtungen umfasst,
wobei eine Wärmetherapievorrichtung (301) umfasst:
- mehrere nach oben vorstehende Akupressur-Noppen (310) an ihrer oberen Oberfläche, wobei jede der Akupressur-Noppen (310) mit supraleitfähigem und im fernen Infrarot emittierendem Material (311) befestigt ist;
- einen vertieften Mittelabschnitt (302);
- wobei beide Seitenabschnitte (305) nach oben gebogen sind, um den nach oben vorstehenden Akupressur-Noppen (310) zu ermöglichen, mit einem Patientenkörper, insbesondere mit dem Rücken oder mit dem Bauch des Patienten, in engen Kontakt zu gelangen;
- Griffe (303, 304), die an jeweiligen oberen Enden der beiden seitlichen Oberflächen der Wärmetherapievorrichtung (301) ausgebildet sind;
- ein Fenster (308) zum digitalen Anzeigen der Temperatur, das auf einer Seite einer oberen Oberfläche der Wärmetherapievorrichtung (301) installiert ist;
- einen oberen und einen unteren Temperatureinstellknopf (309, 309'), die jeweils auf einer Seite des Temperaturanzeigefensters (308) installiert sind;
- eine EIN/AUS-Taste (306) des Wippentyps, die zwischen dem oberen und dem unteren Temperatureinstellknopf (309, 309') vorgesehen ist; und
- eine Leitung (307) für elektrischen Strom, die an einer Seitenoberfläche der Wärmetherapievorrichtung (301) unter einem der Griffe (303) der Wärmetherapievorrichtung (301) ausgebildet ist;
wobei das Wärmetherapiesystem ferner umfasst:
a) einen oberen und einen unteren Körper (A, B), in denen jeweils ein Aufnahmeraum definiert ist;
b) Steuerkästen (C, C'), die an benachbarten Abschnitten des oberen und des unteren Körpers (A, B) ausgebildet sind und dazu ausgelegt sind, jeweilige Komponenten zu steuern;
c) Steuerkonsolen (D, D') in Form einer U-förmigen Platte, die schwenkbar ausgebildet sind, derart, dass jede Steuerkonsole (D, D') auf ihrer einen Seite mit einer Oberseite der jeweiligen Steuerkästen (C, C') über Verbinder (1) verbunden ist, wobei die Steuerkonsolen (D, D') mit Leitungen für elektrischen Strom der Wärmetherapievorrichtungen (3, 4, 110, 110', 211, 211') verbunden sind;
d) Motoren (E, E'), die an einer Mittelposition jeweiliger unterer Abschnitte des oberen bzw. des unteren Körpers (A, B) ausgebildet sind;
e) Riemenscheiben (F, F'), die an jeweiligen gegenüberliegenden Seiten der Motoren (E, E') ausgebildet sind;
f) obere und untere Schienen (10a, 10b, 10'a, 10'b), die zwischen den Motoren (E, E') und den Riemenscheiben (F, F') ausgebildet sind, wobei die oberen und unteren Schienen (10a, 10b, 10'a, 10'b) in zwei Reihen angeordnet sind, um der Wirbelsäule des Körpers zu entsprechen;
g) Synchronriemen (2, 2'), die längs einer Mittelachse zwischen den oberen und unteren Schienen (10a, 10b, 10'a, 10'b) angeordnet sind und direkt mit den Motoren (E, E') bzw. mit den Riemenscheiben (F, F') verbunden sind, wobei jeder Synchronriemen (2, 2') an seiner unteren Oberfläche mit Höckern ausgebildet ist;
h) obere und untere bewegliche Einheiten (G, H), die mit den Synchronriemen (2, 2') gekoppelt sind und auf den oberen und unteren Schienen (10a, 10b, 10'a, 10'b) sitzen, wobei die oberen und unteren beweglichen Einheiten (G, H) mit mehreren Wärmetherapievorrichtungen (110, 110', 211, 211') ausgebildet sind; und
i) zwei andere Wärmetherapievorrichtungen (3, 4), die am oberen Körper (A) ausgebildet sind, wobei die zwei anderen Wärmetherapievorrichtungen (3, 4) auf der linken bzw. auf der rechten Seite der oberen Schienen (10a, 10b) ausgebildet sind und dazu ausgelegt sind, auf beide Arme des Patienten eine Akupressur und eine Wärmebehandlung auszuüben.

2. Wärmetherapiesystem nach Anspruch 1, wobei jede der oberen Schienen (10a, 10b) eine Schiene (11) umfasst, die an ihrem oberen Abschnitt mit mehreren Gewindebohrungen (12) versehen ist, und mehrere abnehmbare gekrümmte Schienenplatten (15) umfasst, die in regelmäßigen Abständen angeordnet sind und an ihren oberen Abschnitten mit mehreren jeweiligen Gewindebohrungen (14) versehen sind,
wobei am oberen Abschnitt der Schiene (11) durch die Gewindebohrungen (14) der jeweiligen Schienenplatten (15) Schrauben (13) befestigt sind.

3. Wärmetherapiesystem (400) nach Anspruch 1, wobei die obere bewegliche Einheit (G) umfasst:
- einen Träger (100) mit einer Zugkraft-Einstellvorrichtung in seinem Mittelabschnitt;
- Kopplungsabschnitte (112), die dazu ausgelegt sind, mit Kopplungsabschnitten (107) gekoppelt zu werden, die an einer entsprechenden Seite von zwei Paaren von Verbindungsstäben (108, 108') gebildet sind, die jeweils im Träger (100) vorgesehen sind, wobei jeder Kopplungsabschnitt (112) an seiner oberen Oberfläche mit einem nach oben verlängerten Vorsprung (111) ausgebildet ist; und
- die Wärmetherapievorrichtungen (110, 110') an ihren beiden seitlichen Oberflächen mit einem Paar Rollen (113) ausgebildet sind,
wobei der Träger (100) umfasst:
-- einen quadratischen Körper (101), der in einem Mittelabschnitt seiner oberen Oberfläche mit einer geradlinigen Kopplungsnut (102) und einem Paar Gewindebohrungen (103) ausgebildet ist;
-- eine Zugkraft-Einstellvorrichtung, die mit der Kopplungsnut (102) des Körpers (101) gekoppelt ist und dazu ausgelegt ist, eine Zugkraft eines der Synchronriemen (2) einzustellen;
-- zwei Paare von Scharnieren (104, 104'), die an beiden seitlichen Oberflächen des Körpers (101) ausgebildet sind;
-- Verbindungsstäbe (108, 108'), wobei jeder Verbindungsstab (108, 108') an beiden Endabschnitten mit perforierten Löchern (105, 106) und an seinem einen Ende mit dem Kopplungsabschnitt (107) ausgebildet ist, wobei eines der perforierten Löcher (106) am Kopplungsabschnitt (107) ausgebildet ist und das andere perforierte Loch (105) verwendet wird, um das Scharnier (104, 104') einzustecken; und
-- Rollen (109, 109'), die an gegenüberliegenden äußeren Seiten der jeweiligen Verbindungsstäbe (108, 108') ausgebildet sind und dazu ausgelegt sind, um die entsprechenden Scharniere (104, 104') angebracht zu werden.

4. Wärmetherapiesystem nach Anspruch 3, wobei die Zugkraft-Einstellvorrichtung umfasst:
- eine erste Zugkraft-Einstelleinrichtung (130), die an einer Seite des Körpers (101) ausgebildet ist;
- eine zweite Zugkraft-Einstelleinrichtung (130'), die an der anderen Seite des Körpers (101) ausgebildet ist, wobei die zweite Zugkraft-Einstelleinrichtung (130') den gleichen Aufbau wie die erste Zugkraft-Einstelleinrichtung (130) hat; und
- eine Zugkraft-Einstellschraube (141), die in den in der ersten und in der zweiten Zugkraft-Einstelleinrichtung (130, 130') ausgebildeten Gewindebohrungen (133, 133') befestigt ist und dazu ausgelegt ist, eine Zugkraft des Synchronriemens (2) einzustellen,
wobei die erste Zugkraft-Einstelleinrichtung (130) umfasst:
-- eine untere Zugkraft-Einstellplatte (131), die mit einer Seite eines oberen Mittelabschnitts des quadratischen Körpers (101) gekoppelt ist;
-- eine obere Zugkraft-Einstellplatte (132), die von einer Kante der unteren Zugkraft-Einstellplatte (131) nach oben verlängert ist und mit einer Zugkraft-Einstellschraubenbohrung (133) ausgebildet ist;
-- Riemenbefestigungsschlitze (134), die an einer Seite eines oberen Mittelabschnitts der unteren Zugkraft-Einstellplatte (131) ausgebildet sind und dazu ausgelegt sind, einen der Synchronriemen (2) zu befestigen;
-- mehrere Befestigungslöcher (135), die an beiden Seiten der Riemenbefestigungsschlitze (134) ausgebildet sind;
-- Bewegungsschlitze (136) benachbart zu den Befestigungslöchern (135), wobei die Bewegungsschlitze (136) so angeordnet sind, dass sie einem Paar der Gewindebohrungen (103), die im Körper (101) ausgebildet sind, entsprechen, um zu ermöglichen, dass Schrauben (137) durch sie hindurch befestigt werden; und
-- eine obere Befestigungsplatte (140), um eine Abtrennung des Synchronriemens (2) zu verhindern, wobei die obere Befestigungsplatte (140) mit mehreren perforierten Löchern (139) ausgebildet ist, um zu ermöglichen, dass Schrauben (138) in den Befestigungslöchern (133) durch sie hindurch befestigt werden.

5. Wärmetherapiesystem nach Anspruch 1, wobei die untere bewegliche Einheit (H) umfasst:
- einen Träger (100'), wobei der Träger (100') umfasst:
-- einen quadratischen Körper (101), der in einem Mittelabschnitt seiner oberen Oberfläche mit einer geradlinigen Kopplungsnut (102) und einem Paar Gewindebohrungen (103) ausgebildet ist;
-- eine Zugkraft-Einstellvorrichtung, die mit der Kopplungsnut (102) des Körpers (101) gekoppelt ist und dazu ausgelegt ist, eine Zugkraft eines der Synchronriemen (2) einzustellen;
-- zwei Paare von Scharnieren (104, 104'), die an beiden seitlichen Oberflächen des Körpers (101) ausgebildet sind;
-- Verbindungsstäbe (108, 108'), wobei jeder Verbindungsstab (108, 108') an beiden Endabschnitten mit perforierten Löchern (105, 106) und an seinem einen Ende mit dem Kopplungsabschnitt (107) ausgebildet ist, wobei eines der perforierten Löcher (106) am Kopplungsabschnitt (107) ausgebildet ist und das andere perforierte Loch (105) verwendet wird, um das Scharnier (104, 104') einzusetzen; und
-- Rollen (109, 109'), die an gegenüberliegenden äußeren Seiten der jeweiligen Verbindungsstäbe (108, 108') ausgebildet sind und dazu ausgelegt sind, um die jeweiligen Scharniere (104, 104') angebracht zu werden;
- einen rechtwinkligen Arm (200), der nach oben vorstehende Abschnitte mit einer bestimmten Länge besitzt, wobei die nach oben vorstehenden Abschnitte mit den Kopplungsabschnitten (107), die an den jeweiligen Verbindungsstäben (108) des in der unteren beweglichen Einheit vorgesehenen Trägers (100') ausgebildet sind, gekoppelt sind;
- ein Paar Rollen (201), die an einer unteren Oberfläche des Arms (200) ausgebildet sind;
- mehrere Gewindebohrungen (202), die geradlinig an beiden Seiten einer oberen Oberfläche des Arms (200) angeordnet sind;
- Schienen (206, 206'), die einen L-förmigen Querschnitt haben, wobei die Schienen (206, 206') mit einer oberen Seite der Gewindebohrungen (202) gekoppelt sind, um eine T-förmige Nut (203) zu bilden, wobei jede Schiene (206, 206') mit perforierten Löchern (205) ausgebildet ist, um zu ermöglichen, dass Schrauben (204) durch sie hindurch in den Gewindebohrungen (202) befestigt werden;
- ein T-förmiges Einsetzelement (208), das dazu ausgelegt ist, in die T-förmige Nut (203) eingesetzt zu werden, die zwischen den Schienen (206, 206') definiert ist, wobei das T-förmige Einsetzelement (208) mit mehreren Bohrungen (207) ausgebildet ist, die längs einer Mittelachse hiervon geradlinig angeordnet sind; und
- Wärmetherapievorrichtungen (211, 211'), die über der T-förmigen Nut (203) angeordnet sind und mit mehreren Gewindebohrungen (209) ausgebildet sind, die den Bohrungen (207) entsprechen, um **dadurch** zu ermöglichen, dass Schrauben (210) durch sie hindurch befestigt werden.

6. Wärmetherapiesystem (400) nach Anspruch 1, wobei jede der Wärmetherapievorrichtungen (110, 110', 210, 210'), die an den oberen und unteren beweglichen Einheiten (G, H) installiert sind, an ihrer oberen Oberfläche mit mehreren nach oben vorstehenden Akupressur-Noppen (310) versehen ist, die mit dem supraleitenden und im fernen Infrarot emittierenden Material (311) befestigt sind; und
wobei die jeweiligen Wärmetherapievorrichtungen (110, 110', 210, 210') nach oben gekrümmt sind, um ihnen zu ermöglichen, mit dem Patientenkörper, insbesondere mit dem Rücken oder mit dem Bauch des Patienten, in engen Kontakt zu gelangen.

7. Wärmetherapiesystem nach Anspruch 1, wobei jede der Wärmetherapievorrichtungen (3, 4), die auf der linken und auf der rechten Seite der oberen Schienen (10a, 10b) installiert sind, an ihrer oberen Oberfläche mit mehreren nach oben vorstehenden Akupressur-Noppen (310) versehen ist, die mit dem supraleitenden und im fernen Infrarot emittierenden Material (311) befestigt sind; und
- wobei die jeweiligen Wärmetherapievorrichtungen (3, 4) nach oben gekrümmt sind, um ihnen zu ermöglichen, mit dem Körper, insbesondere dem Rücken oder dem Bauch des Patienten, in engen Kontakt zu gelangen.

## Revendications

1. Système de thérapie thermique (400) comprenant des dispositifs de thérapie thermique, dans lesquels un dispositif de thérapie thermique (301) comprend :
- une pluralité de plots d'accupressure en projection vers le haut (310) à sa surface supérieure, chacun des plots d'accupressure (310) étant attaché avec un matériau supraconducteur émettant des infrarouges lointains (311) ;
- une portion médiane en creux (302) ;
- les deux portions latérales (305) étant incurvées vers le haut pour permettre aux plots d'accupressure en projection vers le haut (310) de venir en contact intime avec le corps d'un patient, spécialement le dos ou l'abdomen du patient ;
- des poignées (303, 304) formées à des extrémités supérieures respectives des deux surfaces latérales du dispositif de thérapie thermique (301), respectivement ;
- une fenêtre d'affichage de température numérique (308) installée sur un côté d'une surface supérieure du dispositif de thérapie thermique (301) ;
- des boutons d'ajustement de température supérieure et inférieure (309, 309') installés respectivement sur un côté de la fenêtre d'affichage de température (308) ;
- un bouton marche/arrêt (306) du type à bascule, prévu entre les boutons d'ajustement de température supérieure et inférieure (309, 309') ; et
- une ligne de puissance électrique (307) formée sur une surface latérale du dispositif de thérapie thermique (301) sous l'une des poignées (303) du dispositif de thérapie thermique (301) ; le système de thérapie thermique comprenant encore :
a) un corps supérieur et un corps inférieur (A, B) définissant un espace de réception à l'intérieur, respectivement ;
b) des boîtes de commande (C, C') formées à des positions adjacentes du corps supérieur et du corps inférieur (A, B) et adaptées à commander des composants respectifs ;
c) des panneaux de commande (D, B') ayant la forme d'une plaque en U et étant formés de manière incurvée de telle façon que chaque panneau de commande (D, B') est connecté sur un côté à un côté supérieur des boîtes de commande respectives (C, C') au moyen de connecteurs (1), les panneaux de commande (D, B') étant connectés à des lignes de puissance électrique des dispositifs de thérapie thermique (3, 4, 110, 110', 211, 211');
d) des moteurs (E, E') formés à une position centrale des portions inférieures respectives du corps supérieur et du corps inférieur (A, B), respectivement ;
e) des poulies (F, F') formées à des côtés opposés des moteurs (E, E') respectivement ;
f) des rails supérieurs et inférieurs (10a, 10b, 10'a, 10'b) installés entre les moteurs (E, E') et les poulies (F, F') respectivement, les rails supérieurs et inférieurs (10a, 10b, 10'a, 10'b) étant agencés en deux rangées pour correspondre à la colonne vertébrale du corps ;
g) des courroies de synchronisation (2, 2') positionnées le long d'un axe central entre les rails supérieurs et inférieurs (10a, 10b, 10'a, 10'b) tout en étant directement connectées aux moteurs (E, E') et aux poulies (F, F') respectivement, chaque courroie de synchronisation (2, 2') étant formée avec des bossages à sa surface inférieure ;
h) des unités mobiles supérieure et inférieure (G, H) couplées aux courroies de synchronisation (2, 2') respectivement, et logées sur les rails supérieurs et inférieurs (10a, 10b, 10'a, 10'b), les unités mobiles supérieure et inférieure (G, H) étant formées avec une pluralité de dispositifs de thérapie thermique (110, 110', 211, 211') ; et
i) deux autres dispositifs de thérapie thermique (3, 4) formés sur le corps supérieur (A), les deux autres dispositifs de thérapie thermique (3, 4) étant formés sur le côté gauche et le côté droit des rails supérieurs (10a, 10b) et adaptés à appliquer un traitement d'accupressure et un traitement thermique aux deux bras du patient.

2. Système de thérapie thermique selon la revendication 1, dans lequel chacun des rails supérieurs (10a, 10b) comprend un rail (11) formé à sa portion supérieure avec une pluralité de trous à vis (12), et une pluralité de plaques de rails (15) incurvées détachables agencées à distance régulière et formées à leur portion supérieure avec une pluralité de trous à vis (14) respectivement,
grâce à quoi des vis (13) sont fixées sur la portion supérieure du rail (11) à travers les trous à vis (14) des plaques de rails (15), respectivement.

3. Système de thérapie thermique (400) selon la revendication 1, dans lequel l'unité mobile supérieure (G) comprend :
- un support (100) ayant un dispositif d'ajustement de force de traction à une portion centrale de lui-même ;
- des portions de couplage (112) adaptées à être couplées avec des portions de couplage (107) formées sur un côté respectif de deux paires de barres de liaison (108, 108') prévues dans le support (100), respectivement, chaque portion de couplage (112) étant formée avec une projection (111) en extraction vers le haut à sa surface supérieure ; et
- les dispositifs de thérapie thermique (110, 110') formés avec une paire de rouleaux (113) au niveau des deux surfaces latérales, respectivement,
le support (100) comprenant :
-- un corps carré (101) formé au niveau d'une portion centrale de sa surface supérieure avec une gorge de couplage linéaire (102) et une paire de trous à vis (103) ;
-- un dispositif d'ajustement de force de traction couplé à la gorge de couplage (102) du corps (101) et adapté à ajuster une force de traction de l'une des courroies de synchronisation (2) ;
-- deux paires de charnière (104, 104') formées au niveau des deux surfaces latérales du corps (101) ;
-- des barres de liaison (108, 108'), chaque barre de liaison (108, 108') étant formée à ses deux portions terminales avec des trous perforés (105, 106) et à l'une de ses extrémités avec la portion de couplage (107), l'un des trous perforés (106) étant formé au niveau de la portion de couplage (107), l'autre trou perforé (105) étant utilisé pour l'introduction de la charnière (104, 104') ; et
-- des rouleaux (109, 109') formés au niveau de côtés extérieurs opposés des barres de liaison respectives (108, 108') et adaptés à être couplés autour des charnières respectives (104, 104'), respectivement.

4. Système de thérapie thermique selon la revendication 3, dans lequel le dispositif d'ajustement de force de traction comprend :
- un premier élément d'ajustement de force de traction (130) formé à un côté du corps (101) ;
- un second élément d'ajustement de force de traction (130') formé de l'autre côté du corps (101), le second élément d'ajustement de force de traction (130') ayant la même structure que celle du premier élément d'ajustement de force de traction (130) ; et
- une vis d'ajustement de force de traction (141) fixée dans les trous à vis (133, 133') formés au niveau du premier et du second élément d'ajustement de force de traction (130, 130') et adaptée à ajuster une force de traction de la courroie de synchronisation (2),
le premier élément d'ajustement de force de traction (130) comprenant :
-- une plaque d'ajustement de force de traction inférieure (131) couplée à un côté d'une portion centrale supérieure du corps carré (101) ;
-- une plaque d'ajustement de force de traction supérieure (132) qui s'étend vers le haut depuis un bord de la plaque d'ajustement de force de traction inférieure (131) et qui est formée avec le trou à vis pour l'ajustement de force de traction (133) ;
-- des fentes de fixation de courroie (134) formées sur un côté d'une portion centrale supérieure de la plaque d'ajustement de force de traction inférieure (131) et adaptées à fixer l'une des courroies de synchronisation (2) ;
-- une pluralité de trous de fixation (135) formés sur les deux côtés des fentes de fixation de courroie (134) ;
-- des fentes de mouvement (136) adjacentes au trou de fixation (135), les fentes de mouvement (136) étant positionnées pour correspondre avec une paire des trous à vis (103) formés sur le corps (101), permettant ainsi aux vis (137) d'être fixées à travers ceux-ci ; et
-- une plaque de fixation supérieure (140) pour empêcher une coupure de la courroie de synchronisation (2), la plaque de fixation supérieure (140) étant formée avec une pluralité de trous perforés (139) pour permettre aux vis (138) d'être fixées au trou de fixation (133) à travers ceux-ci.

5. Système de thérapie thermique selon la revendication 1, dans lequel l'unité mobile inférieure (H) comprend :
- un support (100'), le support (100') comprenant :
-- un corps carré (101) formé à une portion centrale de sa surface supérieure avec une gorge de couplage linéaire (102) et une paire de trous à vis (103) ;
-- un dispositif d'ajustement de force de traction couplé à la gorge de couplage (102) du corps (101) et adapté à ajuster une force de traction de l'une des courroies de synchronisation (2) ;
-- deux paires de charnières (104, 104') formées au niveau des deux surfaces latérales du corps (101) ;
-- des barres de liaison (108, 108'), chaque barre de liaison (108, 108') étant formée au niveau des deux portions terminales avec des trous perforés (105, 106) et à l'une de ses extrémités avec la portion de couplage (107), l'un des trous perforés (106) étant formé au niveau de la portion de couplage (107), et l'autre trou perforé (105) étant utilisé pour l'introduction de la charnière (104, 104') ; et
-- des rouleaux (109, 109') formés à des côtés extérieurs opposés des barres de liaison respectives (108, 108') et adaptés à être couplés autour des charnières respectives (104, 104'), respectivement ;
- une platine rectangulaire (200) ayant des portions d'une certaine longueur en projection vers le haut, les portions en projection vers le haut étant couplées avec les portions de couplage (107) formées sur les barres de liaison respectives (108) du support (100') prévu dans l'unité mobile inférieure ;
- une paire de rouleaux (201) formés à une surface inférieure de la platine (200) ;
- une pluralité de trous à vis (202) agencés linéairement sur les deux côtés d'une surface supérieure de la platine (200) ;
- des rails (206, 206') ayant une section transversale en forme de L, les rails (206, 206') étant couplés à un côté supérieur des trous à vis (202) pour former une gorge en forme de T (203), chaque rail (206, 206') étant formé avec des trous perforés (205) pour permettre aux vis (204) d'être fixées aux trous à vis (202) à travers ceux-ci ;
- un élément d'insertion en forme de T (208) adapté à être logé dans la gorge en forme de T (203) définie entre les rails (206, 206'), l'élément d'insertion en forme de T (208) étant formé avec une pluralité de trous (207) agencés linéairement le long d'un axe central de celui-ci ; et
- des dispositifs de thérapie thermique (211, 211') disposés au-dessus de la gorge en forme de T (203) et formés avec une pluralité de trous à vis (209) correspondant aux trous (207), permettant ainsi aux vis (210) d'être fixées à travers ceux-ci.

6. Système de thérapie thermique (400) selon la revendication 1, dans lequel chacun des dispositifs de thérapie thermique (110, 110', 210, 210') installés sur les unités mobiles supérieure et inférieure (G, H) est pourvu à sa surface supérieure d'une pluralité de plots d'accupressure (310) en projection vers le haut, qui sont attachés avec le matériau supraconducteur (311) qui émet dans les infrarouges lointains, respectivement ; et
dans lequel les dispositifs de thérapie thermique respectifs (110, 110', 210, 210') sont incurvés vers le haut pour leur permettre de venir en contact intime avec le corps du patient, spécialement le dos ou l'abdomen du patient.

7. Système de thérapie thermique selon la revendication 1, dans lequel chacun des dispositifs de thérapie thermique (3, 4) installés sur les côtés gauche et droit des rails supérieurs (10a, 10b) est pourvu à sa surface supérieure d'une pluralité de plots d'accupressure (310) en projection vers le haut, qui sont attachés avec le matériau supraconducteur (311) qui émet dans les infrarouges lointains, respectivement ; et
- dans lequel les dispositifs de thérapie thermique respectifs (3, 4) sont incurvés vers le haut pour leur permettre de venir en contact intime avec le corps, spécialement le dos ou l'abdomen, du patient.
